# EUROPEAN PATENT APPLICATION

(11) **EP 4 647 878 A1**
(43) Date of publication of application: **12.11.2025**
(21) Application number: 25171642.9
(22) Date of filing: 22.04.2025
(51) Int. Cl.: G06F 3/01

(54) **METHOD FOR MANIPULATING A THREE-DIMENSIONAL IMAGE AND DISPLAY MODULE**

(30) Priority: 07.05.2024 TW 113116856
(71) Applicant: InnoLux Corporation, Jhunan Town Miao li 35053 (TW)
(72) Inventor: LIOU, Hao-Yu, 35053 Jhunan Town, Miaoli County (TW); LU, Wei-Yi, 35053 Jhunan Town, Miaoli County (TW); SUMI, Naoki, 35053 Jhunan Town, Miaoli County (TW); WENG, Ruey-Jer, 35053 Jhunan Town, Miaoli County (TW)
(74) Representative: Becker, Eberhard

(57) **Abstract**

A method for manipulating a three-dimensional image (400) and a display module (100) are provided. A three-dimensional image (400) displays a plurality of objects. Different objects have different defined values. The method for manipulating the three-dimensional image (400) includes the following steps: detecting a position (201) of eyes (200) of a user; determining a first coordinate point (202) according to the position of the eyes; locating the first pointer position (202) on a display (120), and generating a second coordinate point; generating a vector (L1 to L6) according to the first coordinate point and the second coordinate point, wherein the vector (L1 to L6) passes through the plurality of objects; determining a second pointer position on the vector (L1 to L6) according to changes in defined values of the plurality of objects on the vector (L1 to L6); and operating on the object corresponding to the second pointer position.

## Description

### BACKGROUND

### Technical Field

The disclosure relates to a display technique, and in particular, to a method for manipulating a three-dimensional image and a display module.

### Description of Related Art

In the process of displaying three-dimensional image information on a traditional three-dimensional display, if the user needs to select specific spatial position coordinates, he or she usually needs to input complex operating instructions or manually perform related image processing. In other words, for traditional three-dimensional displays, it is difficult for the user to select specific spatial position coordinates in three-dimensional image information and perform subsequent data processing or image editing.

### SUMMARY

The disclosure provides a method for manipulating a three-dimensional image and a display module that may provide a convenient three-dimensional image operation function.

A method for manipulating a three-dimensional image of the disclosure may display a plurality of objects via a three-dimensional image, and different objects have different defined values. The method for manipulating the three-dimensional image includes the following steps: detecting a position of eyes of a user; determining a first coordinate point according to the position of the eyes; locating a first pointer position on a display, and generating a second coordinate point; generating a vector according to the first coordinate point and the second coordinate point, wherein the vector passes through the plurality of objects; determining a second pointer position on the vector according to changes in defined values of the plurality of objects on the vector; and operating on the object corresponding to the second pointer position.

A display module for operating on a three-dimensional image of the disclosure may display a plurality of objects via a three-dimensional image, and different objects have different defined values. The display module includes a display, an eye tracker, and a processor. The display displays the three-dimensional image. The eye tracker detects a position of eyes of a user. The processor is coupled to the display and the eye tracker. The processor determines a first coordinate point according to the position of the eyes. The processor locates the first pointer position on the display to generate a second coordinate point. The processor generates a vector according to the first coordinate point and the second coordinate point. The vector passes through the plurality of objects. The processor determines a second pointer position on the vector according to changes in defined values of the plurality of objects on the vector. The processor operates on the object corresponding to the second pointer position.

Based on the above, the method for manipulating the three-dimensional image and the display module of the disclosure may automatically track the position of the eyes of the user and determine the second pointer position on the corresponding object in the three-dimensional image according to the position of the eyes of the user and the first pointer position in the display screen, so that the user may readily manipulate the corresponding object in the three-dimensional image according to the second pointer position.

In order to make the aforementioned features and advantages of the disclosure more comprehensible, embodiments accompanied with figures are described in detail below.

### BRIEF DESCRIPTION OF THE DRAWINGS

FIG. 1 is a schematic diagram of a display module of an embodiment of the disclosure.
FIG. 2 is a flowchart of a method for manipulating a three-dimensional image of an embodiment of the disclosure.
FIG. 3 is a schematic diagram of manipulating a three-dimensional image of an embodiment of the disclosure.
FIG. 4 is a schematic diagram of image data of a three-dimensional image of an embodiment of the disclosure.
FIG. 5 is a schematic diagram of defined values of image data of an embodiment of the disclosure.
FIG. 6A and FIG. 6B are schematic diagrams of manipulating a three-dimensional image of an embodiment of the disclosure.
FIG. 6C and FIG. 6D are schematic diagrams of manipulating a three-dimensional image of an embodiment of the disclosure.
FIG. 7A is a schematic diagram of manipulating a three-dimensional image of an embodiment of the disclosure.
FIG. 7B is a schematic diagram of defined values of image data of an embodiment of the disclosure.
FIG. 8 is a schematic diagram of image data of a three-dimensional image of an embodiment of the disclosure.
FIG. 9 is a flowchart of an image processing method of an embodiment of the disclosure.

### DESCRIPTION OF THE EMBODIMENTS

Reference will now be made in detail to the exemplary embodiments of the disclosure, and examples of the exemplary embodiments are illustrated in the accompanying drawings. Wherever possible, the same reference numerals are used in the figures and the descriptions to refer to the same or similar portions.

Throughout the disclosure, certain words are used to refer to specific components in the specification and the claims. Those skilled in the art should understand that display equipment manufacturers may refer to the same components by different names. The specification does not intend to distinguish between components having the same function but different names. In the following description and claims, the words "contain" and "include" and the like are open-ended words, and therefore should be interpreted as "including but not limited to..."

In some embodiments of the disclosure, terms related to joining and connecting such as "coupled", "interconnection", etc., unless otherwise defined, may mean that the two structures are in direct contact, or may also mean that the two structures are not in direct contact, wherein there are other structures provided between the two structures. Moreover, terms about joining and connecting may also include the situation where both structures are movable, or both structures are fixed. In addition, the term "coupled" includes any direct and indirect means of electrical connection.

The ordinal numbers used in the specification and claims, such as "first", "second", etc., are used to modify components and do not themselves imply or represent that the component has any previous ordinal number and also do not imply the order of one component relative to another, or the order of manufacturing methods. The use of a plurality of ordinal numbers is used to clearly distinguish a component having a certain name from another component having the same name. The same wording may be not used in the claims and the specification. Accordingly, the first member in the specification may be the second member in the claims. It should be noted that, in the following embodiments, without departing from the spirit of the disclosure, technical features in several different embodiments may be replaced, reorganized, and mixed to complete other embodiments.

The display module of the disclosure may include a virtual reality device, an augmented reality device, a head-up display module, a transparent display module, a sensing device, or a tiling device, but the disclosure is not limited thereto. The display module may be a bendable or flexible electronic device. The display module may be a non-self-luminous display module or a self-luminous display module. The sensing device may be a sensing device for sensing capacitance, light, heat, or ultrasound, but the disclosure is not limited thereto. The display module may, for example, include an electronic element such as a passive element and an active element, such as a capacitor, a resistor, an inductor, a diode, a transistor, etc. The diode may include a light-emitting diode or a photodiode. The light-emitting diode may include, for example, an inorganic light-emitting diode, an organic light-emitting diode (OLED), a mini LED, a micro LED, or a quantum dot LED, but the disclosure is not limited thereto. The tiling device may be, for example, a display tiling device, but the disclosure is not limited thereto. It should be noted that the display module may be any arrangement and combination of the above, but the disclosure is not limited thereto.

It should be noted that, without departing from the spirit of the disclosure, features in several different embodiments may be replaced, reorganized, and mixed to complete other embodiments.

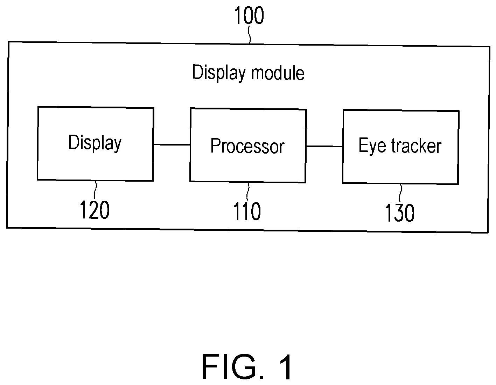

FIG. 1 is a schematic diagram of a display module of an embodiment of the disclosure. Referring to FIG. 1, a display module 100 includes a processor 110, a display 120, and an eye tracker 130. The processor 110 is coupled to the display 120 and the eye tracker 130. In the present embodiment, the display module 100 may be a naked-eye three-dimensional image display device having a three-dimensional image display function. In the present embodiment, the display 120 may display a three-dimensional image, and the eye tracker 130 may track the position of the eyes of the user. The processor 110 may determine the second pointer position on the corresponding three-dimensional object in the three-dimensional image according to the position of the eyes of the user and the first pointer position on the display plane of the display 120, so that the user may manipulate the corresponding three-dimensional object via the second pointer position.

It should be noted that the first pointer position on the display plane of the display 120 may be the coordinates of the corresponding position of the display surface of the display panel of the display 120, but the disclosure is not limited thereto. In an embodiment, the display plane of the display 120 may be parallel to the display surface of the display panel of the display 120. The display plane of the display 120 may also refer to the background plane or the virtual reference plane in the three-dimensional image.

In the present embodiment, the processor 110 may include, for example, a central processing unit (CPU), a graphic processing unit (GPU), or other programmable general-purpose or special-purpose microprocessors, digital signal processors (DSPs), application-specific integrated circuits (ASICs), programmable logic devices (PLDs), or other similar processing circuits, or a combination of the devices.

In the present embodiment, the display 120 may include, for example, a liquid crystal or a light-emitting diode. The light-emitting diode may include, for example, an organic light-emitting diode (OLED), a mini LED, a micro LED, a quantum dot LED, fluorescence, phosphor, or other suitable materials, and the materials thereof may be arbitrarily arranged and combined, but the disclosure is not limited thereto.

In the present embodiment, the eye tracker 130 may include an image sensing element having a depth information sensing function. The eye tracker 130 may be used to determine the eyes of the user, and may locate the position of the eyes of the user in space. In the present embodiment, the position of the eyes may refer to the midpoint position of the line connecting the centers of the two eyes of the user or may be the midpoint position between the eyebrows, but the disclosure is not limited thereto.

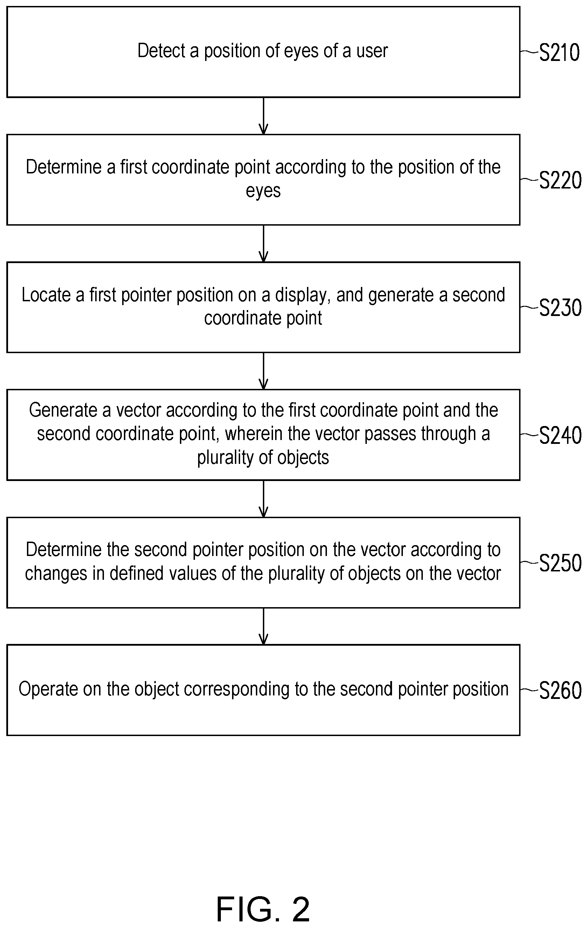

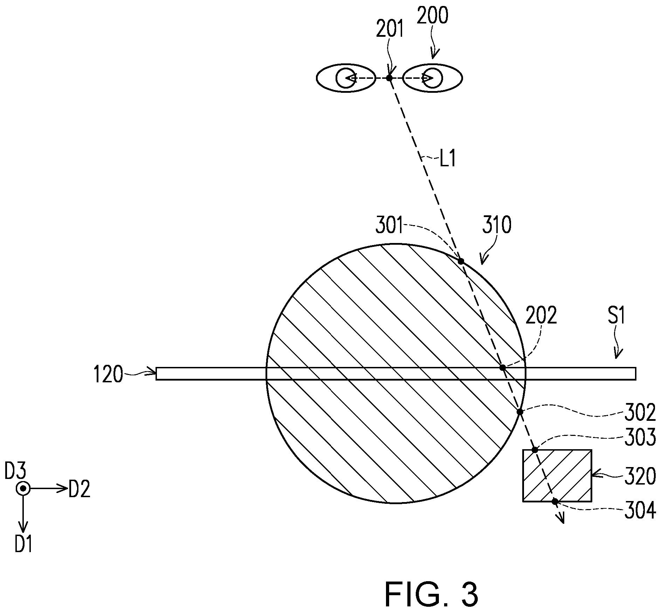

FIG. 2 is a flowchart of a method for manipulating a three-dimensional image of an embodiment of the disclosure. FIG. 3 is a schematic diagram of manipulating a three-dimensional image of an embodiment of the disclosure. Referring to FIG. 1 to FIG. 3, the display module 100 may perform the following steps S210 to S260 to manipulate a three-dimensional image. First, please refer to FIG. 3. FIG. 3 simulates the display effect of a three-dimensional image based on the viewing angle of the user. As shown in FIG. 3, eyes 200 of the user may view the three-dimensional image displayed by the display 120 in a direction D1, and may see that the three-dimensional image displayed by the display 120 may include virtual objects 310 and 320, for example. A screen reference plane S1 of the display 120 may be parallel to a direction D2 and a direction D3, wherein the screen reference plane S1 may refer to the display plane of the display 120.

In addition, the three-dimensional image may be, for example, a result displayed by the processor 110 according to volume data, and the volume data may be composed of, for example, a plurality of layers of two-dimensional medical image data. In an embodiment, the volume data may also be image data of other application fields. In addition, the two-dimensional medical image data may be, for example, a computed tomography (CT) image, a magnetic resonance imaging (MRI) image, or an automated breast ultrasound system (ABUS) image, etc.

In step S210, the eye tracker 130 may detect a position 201 of the eyes 200 of the user. The position 201 of the eyes is the center point between the eyes of the user. In step S220, the processor 110 may determine a first coordinate point according to the position 201 of the eyes. The processor 110 may obtain a first coordinate parameter of the position 201 of the eyes according to a detection result of the eye tracker 130. In step S230, the processor 110 may locate a first pointer position 202 on the display 120 and generate a second coordinate point. The processor 110 may obtain a second coordinate point of the first pointer position 202. In the present embodiment, the first pointer position 202 on the display 120 may be, for example, the position of the cursor in the screen reference plane S1, but the disclosure is not limited thereto. The first pointer position 202 may also be a system-default pointer position. In this regard, the first pointer position 202 may be automatically determined by the processor 110 or determined by the operation result of an input device, for example. The input device may be, for example, a mouse, a laser pen, a touch panel, an infrared light sensing module, a Time-of-Flight (ToF) sensing module, a structured light sensing module, or a related planar input device, but the disclosure is not limited thereto.

In step S240, the processor 110 may generate a vector L1 according to the first coordinate point and the second coordinate point, wherein the vector L1 passes through the objects 310 and 320, for example. The processor 110 may define the vector L1 according to the line connecting the position 201 of the eyes to the first pointer position 202. In step S250, the processor 110 may determine the second pointer position on the vector L1 according to changes in the defined values of the objects 310 and 320 on the vector L1. In the present embodiment, the changes in the defined values may be, for example, changes in accumulated defined values or gradient changes in accumulated defined values, but the disclosure is not limited thereto. In step S260, the processor 110 may operate on the object corresponding to the second pointer position.

In the present embodiment, the objects 310 and 320 presented in the display screen are composed of pixels, and each pixel is defined with a different color, chroma, brightness, gray scale, transparency, or saturation according to the desired display screen data, thereby presenting the display screen and the objects 310 and 320 in the screen thereof. For example, the three primary colors (red, blue, and green) and transparency are all divided into 256 gradients (i.e., 0 to 255), the surface of the object 310 may be defined as red 255, blue 0, green 0, transparency 0, and the interior of the object 310 may be defined as red 255, blue 0, green 0, transparency 128, then the surface of the object 310 is rendered as a red opaque surface, and the interior of the object 310 is rendered as a red translucent surface, but the disclosure is not limited thereto. The defined values in the disclosure may be, for example, the defined pixel values of an object in the display screen, so the changes in the defined values may include changes in the pixel values, but the disclosure is not limited thereto. For example, the surfaces of the objects 310 and 320 may, for example, have lower pixel values (in terms of transparency as described above). Therefore, the processor 110 may determine whether the pixel value is lower than a preset pixel value or has a greater pixel value difference with an adjacent pixel to determine the object boundary. As shown in FIG. 3, along the vector L1, there may be feature points 301 to 304 between the vector L1 and the object boundaries of the objects 310 and 320 (that is, the intersection points of the vector L1 and the object boundaries). In this regard, the processor 110 may select one of the feature points 301 to 304 according to the operation of the user of a mouse or other input element, and display the second pointer on the selected feature point. In this way, the user may effectively select a point of one of the surfaces of the objects 310 and 320 (i.e., for example, one of the feature points 301 to 304) to perform the relevant manipulation function. In some embodiments, the second pointer may be displayed adjacent to the feature point, that is, the second pointer may be a distance away from the feature point. For example, the second pointer may be displayed inside the object 310 but adjacent to the feature point, but the disclosure is not limited thereto.

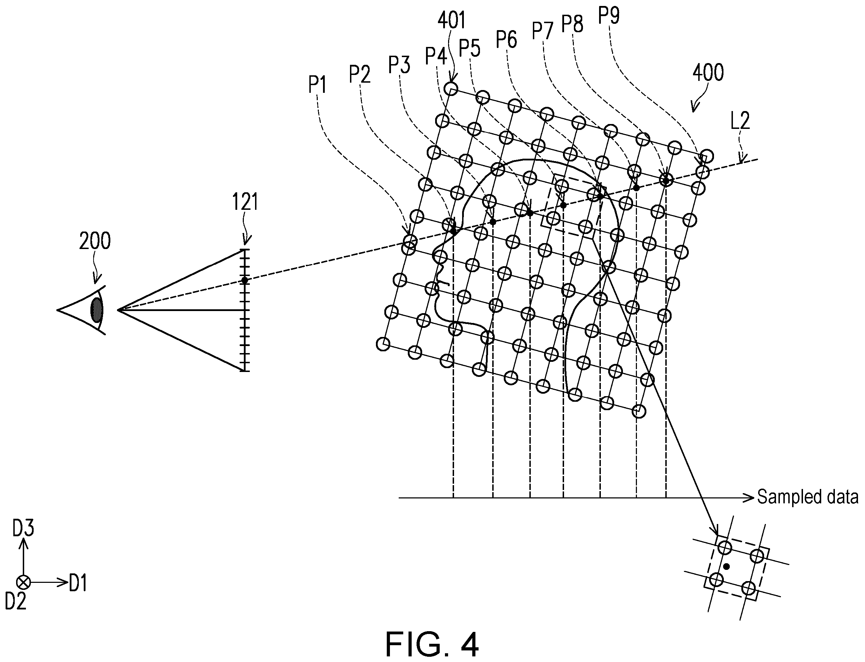

FIG. 4 is a schematic diagram of image data of a three-dimensional image of an embodiment of the disclosure. Referring to FIG. 1 and FIG. 4, the eyes 200 of the user can, for example, view a three-dimensional image 400 via a display plane 121 of the display 120, wherein the three-dimensional image 400 may be, for example, a three-dimensional medical image generated based on volume data. As shown in FIG. 4, the three-dimensional image 400 may be, for example, a skull image. The three-dimensional image 400 may be composed of a plurality of voxels 401 (or volume pixels), and each voxel may have corresponding volume data. In the present embodiment, the volume data may include, for example, a radiation absorption value or a magnetic field release time.

In the present embodiment, the processor 110 may determine a vector L2 according to the method of the above embodiment, and the processor 110 may determine a plurality of locations on the vector L2 according to the distance change or the depth change of the object on the vector L2. As shown in FIG. 4, the processor 110 may define that the vector L2 has a plurality of points P1 to P9 on the path through the three-dimensional image 400, wherein the points P1 and P9 may be two points on the surface of the three-dimensional image 400 respectively, and the points P2 to P8 are equidistant between the points P1 and P9.

In the present embodiment, the point P8 may correspond to the original data of a certain voxel, and the points P1 to P7 and P9 may respectively correspond to the respective sampled data thereof, wherein the sampled data may be generated by interpolation from the original data. In the present embodiment, the original data may include a CT value (HU), but the disclosure is not limited thereto. As shown in FIG. 4, the position P5 may be generated by interpolating the original data of a plurality of adjacent voxels.

In the present embodiment, the processor 110 may automatically display the points P1 to P9 for the user to select via the input device, so that one of the points P1 to P9 may be selected as the second pointer position. That is, the coordinates of one of the points P1 to P9 may be the coordinates of the second pointer position. In this way, the user can, for example, read the corresponding sampled data or original data according to the second pointer position, or perform a relevant operation such as image editing or image processing on the three-dimensional image 400 based on the second pointer position, but the disclosure is not limited thereto.

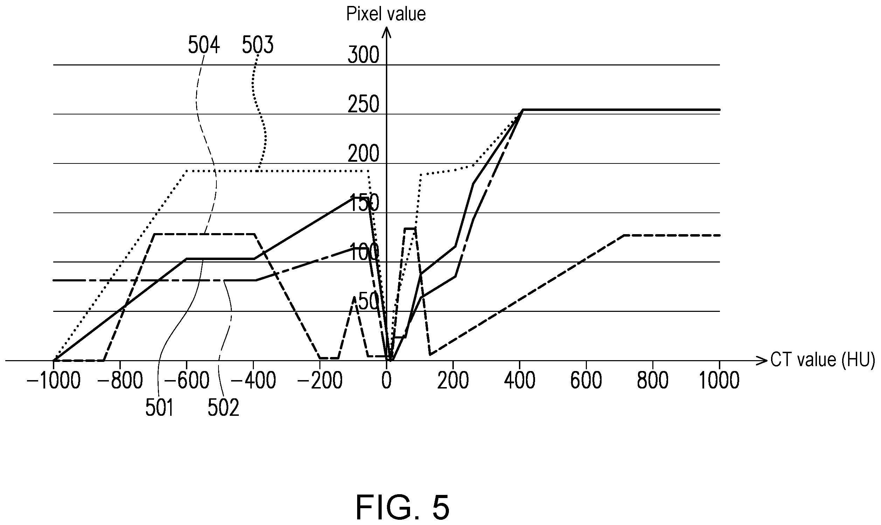

FIG. 5 is a schematic diagram of defined values of image data of an embodiment of the disclosure. Referring to FIG. 1 and FIG. 5, the defined values may also be red pixel values, green pixel values, blue pixel values, and transparency. The above defined values may be, for example, divided into 256 gradients, that is, 0 to 255, but the disclosure is not limited thereto. The processor 110 may also render the CT values in the volume data via image rendering according to the numerical relationship shown in FIG. 5 to generate corresponding display data, wherein a curve 501 may be the relationship between red pixel values and CT values, a curve 502 may be the relationship between green pixel values and CT values, a curve 503 may be the relationship between blue pixel values and CT values, and a curve 504 may be the relationship between transparency and CT values, but the disclosure is not limited thereto. The processor 110 may convert the volume data into a three-dimensional image having color according to the numerical relationship shown in FIG. 5.

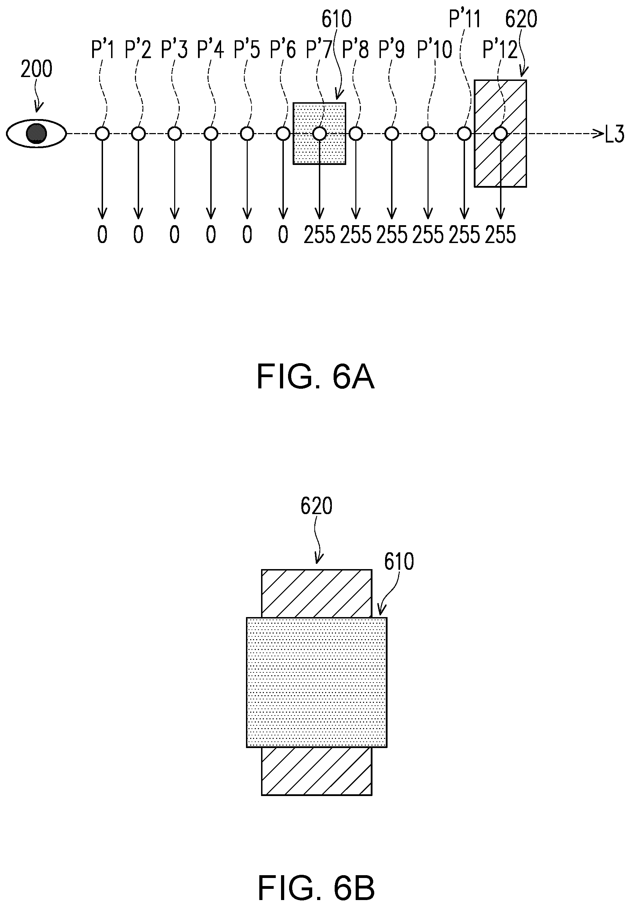

FIG. 6A and FIG. 6B are schematic diagrams of manipulating a three-dimensional image of an embodiment of the disclosure. Referring to FIG. 1 and FIG. 6A, the processor 110 may determine a vector L3 according to the method of the above embodiment, wherein the vector L3 is extended from the eyes 200 of the user to the display 120. On the path of the vector L3, the vector L3 may, for example, pass through a plurality of points P1 to P12 of an object in the three-dimensional image. Each point may correspond to the original data or the sampled data of a certain voxel. Each point is rendered according to the original data or the sampled data of the voxel, so that each point is defined by a defined value to generate visual feature points. Feature points P'1 to P'12 may, for example, be represented by a pixel value (such as transparency) corresponding to the image transmittance (that is, if the pixel value (such as transparency) is "0", the image transmittance is "100". On the contrary, if the pixel value (e.g., transparency) is "255", the image transmittance is "0"), but the disclosure is not limited thereto.

In the present embodiment, the vector L3 may pass through an object surface 610 at the position of the feature point P'7, and pass through an object surface 620 at the position of the feature point P'12. In the present embodiment, the accumulation result of the defined values of the feature points P'1 to P'6 is "0", and the accumulation result of the defined values of the feature points P'7 to P'12 is "255". The processor 110 may determine the second pointer position on the vector L3 according to changes in the defined values of the objects on the vector L3.

In the present embodiment, the processor 110 may preset the accumulation threshold of the defined values to "255", but the disclosure is not limited thereto. The processor 110 may determine whether the accumulation result of the defined value of the currently accumulated feature point is equal to or greater than the accumulation threshold, so as to determine that the currently accumulated feature point is the second pointer position.

Specifically, as shown in FIG. 6A, the processor 110 may start accumulating from the defined value of the feature point P'1, and when accumulating to the feature point P'7, the accumulation result of the defined value is "255". Therefore, the processor 110 may define the feature point P'7 as the second pointer position. The processor 110 may stop continuing to accumulate the defined values of the feature points P'8 to P'12. As shown in FIG. 6B, the object surface 610 has a defined value of "255" (i.e., the image transmittance is "0"). Therefore, the object surface 610 displayed by the processor 110 via the display 120 covers the object surface 620, and the portion of the object surface 620 overlapped with the object surface 610 may be completely obscured. Furthermore, the processor 110 may define the feature point P'7 as the second pointer position. In this way, the user can, for example, intuitively select the feature point P'7 as the second pointer position via the input device, and perform subsequent image editing or a related application on the feature point P'7 on the object surface 610.

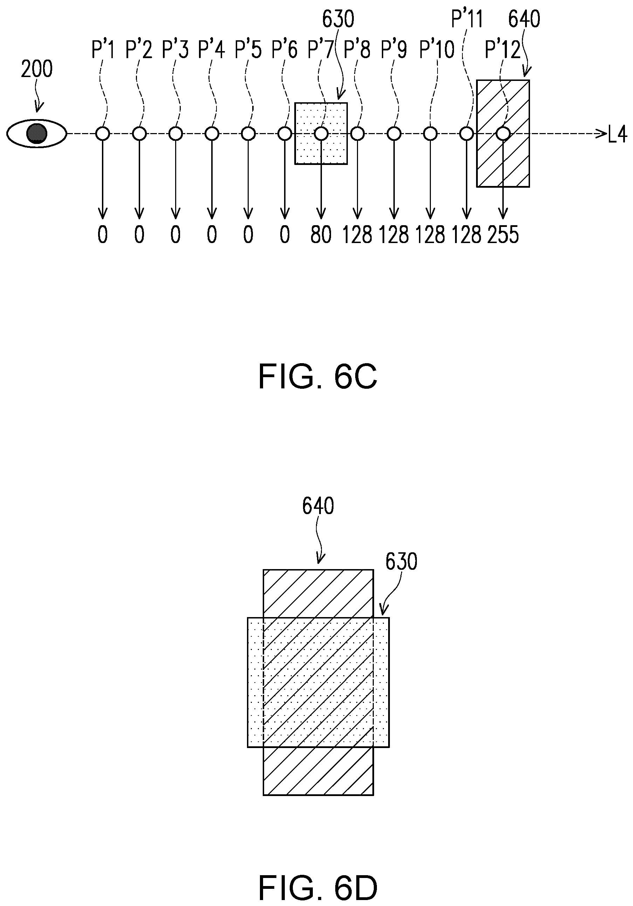

FIG. 6C and FIG. 6D are schematic diagrams of manipulating a three-dimensional image of an embodiment of the disclosure. Referring to FIG. 1 and FIG. 6C, the processor 110 may determine a vector L4 according to the method of the above embodiment, wherein the vector L4 is extended from the eyes 200 of the user to the display 120. On the path of the vector L4, the vector L4 may, for example, pass through the plurality of points P1 to P12 of an object in the three-dimensional image (see FIG. 4). Each point may correspond to the original data or the sampled data of a certain voxel. Each point is rendered according to the original data or the sampled data of the voxel, so that each point is defined by a defined value to generate visual feature points. The feature points P'1 to P'12 may, for example, be represented by a pixel value (such as transparency) corresponding to the image transmittance (that is, if the pixel value (such as transparency) is "0", the image transmittance is "100". On the contrary, if the pixel value (e.g., transparency) is "255", the image transmittance is "0"), but the disclosure is not limited thereto.

In the present embodiment, the vector L4 may pass through an object surface 630 at the position of the feature point P'7, and pass through an object surface 640 at the position of the feature point P'12. In the present embodiment, the accumulation result of the defined values of the feature points P'1 to P'6 is "0", the accumulation result of the defined values of the feature point P'7 is "80", the accumulation result of the defined values of the feature points P'8 to P'11 is "128", and the accumulation result of the defined values of the feature point P'12 is "255". The processor 110 may determine the second pointer position on the vector L4 according to changes in the defined values of the objects on the vector L4.

In the present embodiment, the processor 110 may preset the accumulation threshold of the defined values to "256", but the disclosure is not limited thereto. The processor 110 may determine whether the accumulation result of the defined value of the currently accumulated feature point is equal to or greater than the accumulation threshold, so as to determine that the currently accumulated feature point is the second pointer position.

Specifically, as shown in FIG. 6D, the processor 110 may start accumulating from the defined value of the feature point P'1, and when accumulating to the feature point P'12, the accumulation result of the defined value is "255". Therefore, the processor 110 may define the feature point P'12 as the second pointer position. As shown in FIG. 6D, the object surface 630 has a defined value of "255" (i.e., the image transmittance is "0"). Therefore, the object surface 630 displayed by the processor 110 via the display 120 covers the object surface 640, and the user may see through the portion of the object surface 640 overlapped with the object surface 630. Furthermore, the processor 110 may define the feature point P'12 as the second pointer position. In this way, the user can, for example, intuitively select the feature point P'12 as the second pointer position via the input device, and perform subsequent image editing or a related application on the feature point P'12 on the object surface 640.

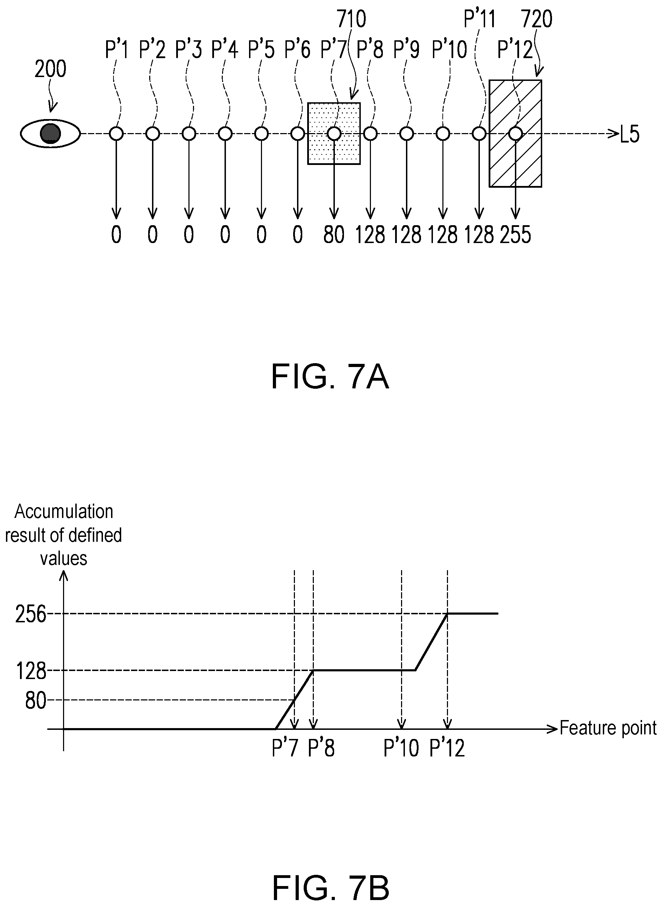

FIG. 7A is a schematic diagram of manipulating a three-dimensional image of an embodiment of the disclosure. Referring to FIG. 1 and FIG. 7A, the processor 110 may determine a vector L5 according to the method of the above embodiment, wherein the vector L5 is extended from the eyes 200 of the user to the display 120. On the path of the vector L5, the vector L5 may, for example, pass through the plurality of points P1 to P12 of the object in the three-dimensional image (see FIG. 4). Each point may correspond to the original data or the sampled data of a certain voxel. Each point is rendered according to the original data or the sampled data of the voxel, so that each point is defined by a defined value to generate visual feature points. The feature points P'1 to P'12 may, for example, be represented by a pixel value (such as transparency) corresponding to the image transmittance (that is, if the pixel value (such as transparency) is "0", the image transmittance is "100". On the contrary, if the pixel value (e.g., transparency) is "255", the image transmittance is "0"), but the disclosure is not limited thereto.

In the present embodiment, the vector L5 may pass through an object surface 710 at the position of the feature point P'7, and pass through an object surface 720 at the position of the feature point P'12. In the present embodiment, the accumulation result of the defined values of the feature points P'1 to P'6 is "0", the accumulation result of the defined value of the feature point P'7 is "80", the accumulation result of the defined values of the feature points P'8 to P'11 is "128", and the accumulation result of the defined values of the feature point P'12 is "255". The processor 110 may determine the second pointer position on the vector L5 according to the gradient changes in the defined values of the objects on the vector L5.

In the present embodiment, the processor 110 may preset one threshold of the gradient changes in the accumulated defined values, but the disclosure is not limited thereto. The processor 110 may determine whether the gradient change in the accumulated defined values of the currently accumulated feature point is equal to or greater than the threshold of the gradient change in the accumulated defined values, so as to determine the currently accumulated feature point as the second pointer position.

Specifically, as shown in FIG. 7B, the processor 110 may start accumulating from the defined value of the feature point P'1, and when accumulating to the feature point P'7, the accumulation result of the defined value is changed from "0" to "80", indicating the feature point P'7 has a greater gradient change in accumulated defined values. In addition, the processor 110 may continue to accumulate from the defined value of the feature point P'7, and when accumulating to the feature point P'8, the accumulation result of the defined value is changed from "80" to "128", indicating the feature point P'8 has another greater gradient change in accumulated defined values. In addition, the processor 110 may continue to accumulate from the defined value of the feature point P'8, and when accumulating to the feature point P'12, the accumulation result of the defined value is changed from "128" to "255", indicating the feature point P'12 has yet another greater gradient change in accumulated defined values.

Therefore, the processor 110 may provide the feature points P'1, P'7, and P'12 for the user to select. One of the feature points P'1, P'7, and P'12 may be selected by the user as the second pointer position. In this way, the user can, for example, intuitively select one of the feature points P'1, P'7, and P'12 as the second pointer position via the input device, and perform subsequent image editing or a related application on one of the feature points P'1, P'7, and P'12.

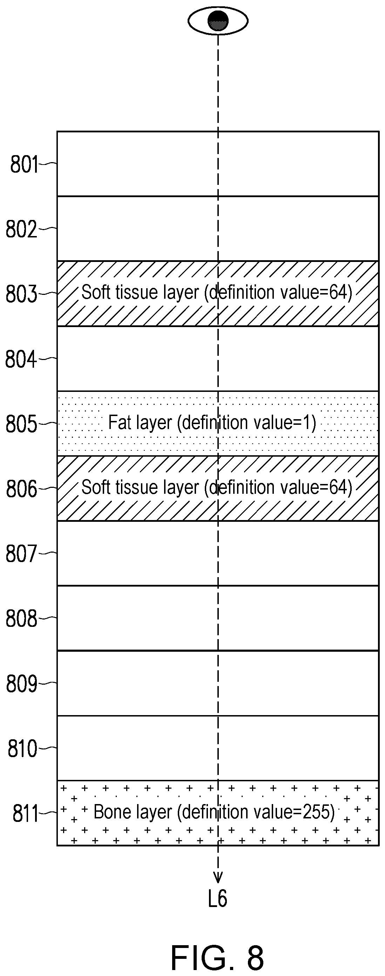

FIG. 8 is a schematic diagram of image data of a three-dimensional image of an embodiment of the disclosure. Referring to FIG. 1 and FIG. 8, the three-dimensional image may convert the CT values of the medical image data into defined values via, for example, the image rendering described in the embodiment of FIG. 5. In the present embodiment, the processor 110 may determine a vector L6 according to the method of the above embodiment, wherein the vector L6 is extended from the eyes 200 of the user to the display 120. On the path of the vector L6, the vector L6 may, for example, pass through a plurality of structural layers 801 to 811 in the three-dimensional image. As shown in FIG. 8, the structural layers 801, 802, 804, 807 to 810 may be air layers, the structural layers 803 and 806 may be soft tissue layers, the structural layer 805 may be a fat layer, and the structural layer 811 may be a bone layer. In the original three-dimensional medical image, the CT values of the structural layers 801, 802, 804, 807 to 810 may be, for example, "-1000", the CT values of the structural layers 803 and 806 may be, for example, "50", the CT value of structural layer 805 may be, for example, "-100", and the CT value of structural layer 811 may be, for example, "400".

As shown in FIG. 8, the defined values of a plurality of corresponding feature points of the structural layers 801, 802, 804, 807 to 810 through which the vector L6 passes may be "0", the defined values of a plurality of corresponding feature points of the structural layers 803 and 806 through which the vector L6 passes may be "64", the defined values of a plurality of corresponding feature points of the structural layer 805 through which the vector L6 passes may be "1", and the defined values of the plurality of corresponding feature points of the structural layer 811 through which the vector L6 passes may be "255". In this regard, the processor 110 may, for example, display a plurality of options corresponding to the structural layers 803, 805, 806, 811 having a defined value greater than 1 for the user to select one of the above as the second pointer position, so that the user may intuitively select the second pointer position via the input device and perform subsequent image editing or a related application on the image data corresponding to the second pointer position.

In an embodiment, the processor 110 may also select the second pointer position according to the difference in defined values or the gradient change in defined values between two adjacent layers of the structural layers 801 to 811, without being limited to the above implementation method.

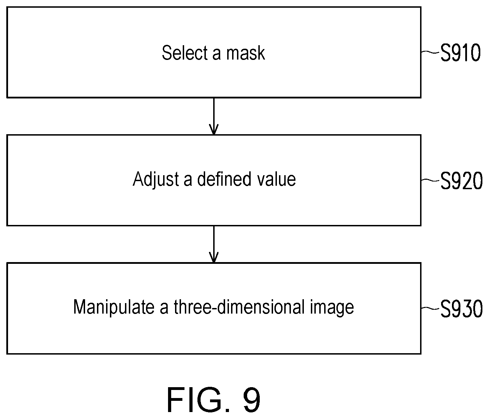

FIG. 9 is a flowchart of an image processing method of an embodiment of the disclosure. Referring to FIG. 1 and FIG. 9, the processor 110 may also edit at least one defined value of at least one object in the three-dimensional image according to at least one mask. In the present embodiment, the processor 110 may perform the following steps S910 to S930 to pre-process the image data of the three-dimensional image. In step S910, the processor 110 may select a mask. In the present embodiment, the mask may refer to an image mask used to select a specific type of object in the three-dimensional image. In step S920, the processor 110 may adjust the defined value of the selected object. As shown in FIG. 8, the processor 110 may select the structural layers 803, 805, 806, and 811 via different masks respectively, and perform image editing to set specific defined values thereof respectively. In step S930, the processor 110 may operate the three-dimensional image. The processor 110 may perform the related manipulation of the three-dimensional image as described in the above embodiments. Therefore, the display module 100 may effectively display the object in the three-dimensional image, and allow the user to conveniently select the second pointer position.

In addition, in an embodiment, the pre-processing of image data may also be implemented by other external computing devices, and is not limited to being processed by the processor 110.

Based on the above, the method for manipulating the three-dimensional image and the display module of the disclosure may automatically track the position of the eyes of the user and define a virtual vector according to the position of the eyes of the user and the first pointer position in the display screen and determine the corresponding second pointer position according to the feature points of the three-dimensional image passed by the virtual vector. In this way, the user may readily control the three-dimensional image according to the second pointer position.

## Claims

1. A method for manipulating a three-dimensional image (400), wherein the three-dimensional image (400) displays a plurality of objects, and different objects have different defined values, wherein the method for manipulating the three-dimensional image (400) comprises:
detecting a position (201) of eyes (200) of a user;
determining a first coordinate point according to the position (201) of the eyes (200);
locating a first pointer position (202) on a display, and generating a second coordinate point;
generating a vector (L1 to L6) according to the first coordinate point and the second coordinate point, wherein the vector (L1 to L6) passes through the objects;
determining a second pointer position on the vector (L1 to L6) according to changes in defined values of the objects on the vector (L1 to L6);
operating on the object corresponding to the second pointer position.

2. The method for manipulating the three-dimensional image (400) of claim 1, wherein the changes in the defined values are changes in accumulated defined values.

3. The method for manipulating the three-dimensional image (400) of claim 1, wherein the changes in the defined values are gradient changes in accumulated defined values.

4. The method for manipulating the three-dimensional image (400) of claim 1, wherein the second pointer position corresponds to original data or sampled data, wherein the sampled data is generated according to an interpolation operation of the original data.

5. The method for manipulating the three-dimensional image (400) of claim 1, further comprising:
editing at least one defined value of the objects in the three-dimensional image (400) according to at least one mask.

6. The method for manipulating the three-dimensional image (400) of claim 5, wherein the at least one mask is used to select at least one object of at least one specific type in the three-dimensional image (400).

7. The method for manipulating the three-dimensional image (400) of claim 1, wherein the three-dimensional image (400) is a display result of volume data, and the volume data is composed of a plurality of layers of two-dimensional medical image data.

8. The method for manipulating the three-dimensional image (400) of claim 1, wherein the first pointer position (202) is a position of a cursor on a screen reference plane (S1) of the display (120).

9. The method for manipulating the three-dimensional image (400) of claim 1, wherein the first pointer position (202) is a system-default pointer position.

10. The method for manipulating the three-dimensional image (400) of claim 1, wherein the position of the eyes is a center point between two eyes of the user.

11. A display module (100) for operating on a three-dimensional image (400), wherein the three-dimensional image (400) displays a plurality of objects, and different objects have different defined values, wherein the display module (100) comprises:
a display (120) displaying the three-dimensional image (400);
an eye tracker (130) detecting a position (201) of eyes (200) of a user; and
a processor (110) coupled to the display (120) and the eye tracker,
wherein the processor (110) determines a first coordinate point according to the position (201) of the eyes (200), and the processor (110) locates a first pointer position (202) on the display (120) to generate a second coordinate point,
wherein the processor (110) generates a vector (L1 to L6) according to the first coordinate point and the second coordinate point, wherein the vector (L1 to L6) passes through the objects,
wherein the processor (110) determines a second pointer position on the vector (L1 to L6) according to changes in defined values of the objects on the vector (L1 to L6), and the processor (110) operates on the object corresponding to the second pointer position.

12. The display module (100) of claim 11, wherein the changes in the defined values are changes in accumulated defined values.

13. The display module (100) of claim 11, wherein the changes in the defined values are gradient changes in accumulated defined values.

14. The display module (100) of claim 11, wherein the second pointer position corresponds to original data or sampled data, wherein the sampled data is generated according to an interpolation operation of the original data.

15. The display module (100) of claim 11, wherein the processor (110) edits at least one defined value of the objects in the three-dimensional image (400) according to at least one mask.
